# EUROPÄISCHE PATENTSCHRIFT

(11) **EP 1 605 845 B1**
(45) Veröffentlichungstag und Bekanntmachung des Hinweises auf die Patenterteilung: **18.08.2010**
(21) Anmeldenummer: 03816329.1
(22) Anmeldetag: 21.03.2003
(51) Int. Cl.: A61B 17/72

(54) **INTRAMEDULLÄRER MARKNAGEL**
INTRAMEDULLARY NAIL
CLOU CENTROMEDULLAIRE

(43) Veröffentlichungstag der Anmeldung: 21.12.2005
(73) Patentinhaber: Synthes GmbH, 4436 Oberdorf (CH)
(72) Erfinder: SCHLIENGER, André, CH-4142 Münchenstein (CH); BUETTLER, Markus, CH-4717 Mümliswil (CH); SENN, Peter, CH-4437 Waldenburg (CH)
(74) Vertreter: Lusuardi, Werther
(86) Internationale Anmeldenummer: PCT/CH2003/000184
(87) Internationale Veröffentlichungsnummer: WO 2004/082494

(56) Entgegenhaltungen:
- DE-A- 19 945 611
- US-A- 5 549 610
- US-A- 6 126 661
- US-A1- 2002 173 792

## Beschreibung

Die Erfindung betrifft einen intramedullären Marknagel gemäss dem Oberbegriff des Patentanspruchs 1, insbesondere für die distale Tibia.

Aus dem Stand der Technik sind bereits Marknägel bekannt, welche auf der gleichen Höhe zwei sich schneidenden Querbohrungen aufweisen, damit der gleiche Marknagel sowohl für einen rechten wie für einen linken Röhrenknochen verwendbar ist.

Die Nachteile dieser bekannten Anordnungen bestehen darin, dass die beiden (oder auch mehrere) Bohrungen sich schneiden und ihr Achsenschnittpunkt auf der Nagellängsachse zu liegen kommt, so dass relativ viel Material aus dem Querschnitt des Marknagels herausgenommen wird, was zu einer erheblichen mechanischen Schwächung des Marknagel an dieser Stelle führt.

Aus der US-A-5 549 610 ist ein modularer Kopfteil für einen intramedullären Femurnagel bekannt der auf dem Oberbegriff des Anspruchs 1 basiert. Die insgesamt fünf Querbohrungen im modularen Kopfteil liegen alle in einer Ebene, welche die Längsachse des modularen Kopfteils beinhaltet. Aus diesem Grund beschränkt sich die Anwendbarkeit der Querbohrungen auf die Fixierung von Femurhals-Frakturen.

Hier will die Erfindung Abhilfe schaffen. Der Erfindung liegt das Problem zugrunde, einen Marknagel mit zwei (oder mehreren) sich kreuzenden Querbohrungen zu schaffen, der eine verbesserte mechanische Festigkeit im Vergleich zum oben beschriebenen Stand der Technik aufweist, ohne dass sein Querschnitt dazu vergrössert werden muss.

Die Erfindung löst die gestellte Aufgabe mit einem Marknagel, welcher die Merkmale des Anspruchs 1 aufweist.

Eine Variante besteht darin, dass sich die Zylinderachsen der beiden sich durchdringenden Bohrungszylinder in einem Schnittpunkt P schneiden, der einen Abstand d > 0 von der Längsachse aufweist. Vorteilhafterweise liegt der Abstand d relativ zum Durchmesser D des Marknagels im Bereich 0,0001 D < d < 0,6000 D, vorzugsweise im Bereich von 0,2 D < d < 0,5 D.

Bei einer weiteren Ausführungsform schneiden sich die Zylinderachsen der beiden sich durchdringenden Bohrungszylinder unter einem Winkel β, wobei 30° < β < 90°, und vorzugsweise 50° < β < 80° gilt. Die optimale Winkelauswahl ergibt eine Optimierung der Wandstärke des Marknagels.

Bei einer bevorzugten Ausführungsform weisen die sich durchdringenden Bohrungszylinder zwei getrennte Eingänge in den Marknagel, aber nur einen gemeinsamen Ausgang aus dem Marknagel auf. Der Vorteil gegenüber 4 Öffnungen liegt im möglichst geringen Materialverlust, d.h. einer höheren Festigkeit des Marknagels.

Der Durchmesser D_{B} der sich durchdringenden Bohrungszylinder beträgt vorteilhafteniveise höchsten das 0,6-fache, vorzugsweise höchstens das 0,5-fache von D.

Bei einer weiteren Ausführungsform weist der Marknagel eine zur zentralen Längsachse koaxiale Längsbohrung auf. Je grösser der Durchmesser dieser Kannulierung relativ zu den Durchmessern der Bohrungszylinder ist, um so angulierter können die sich durchdringenden Querbohrungen zueinander stehen, ohne dass die Querschnittsfläche des Marknagels beeinträchtigt wird d.h. ohne dass die Festigkeit des Marknagels zu reduziert wird.

Die Erfindung und Weiterbildungen der Erfindung werden im Folgenden anhand der teilweise schematischen Darstellungen mehrerer Ausführungsbeispiele noch näher erläutert.

Es zeigen:
Fig. 1 eine Vorder- und Hinteransicht des Marknagels mit zwei sich kreuzenden Querbohrungen;
Fig. 2 eine Vorderansicht des Marknagels mit schematischer, perspektivischer Ansicht der Bohrungszylinder; und
Fig. 3 einen orthogonalen Querschnitt durch den Marknagel nach Fig. 2 im Bereich der sich kreuzenden Querbohrungen; und

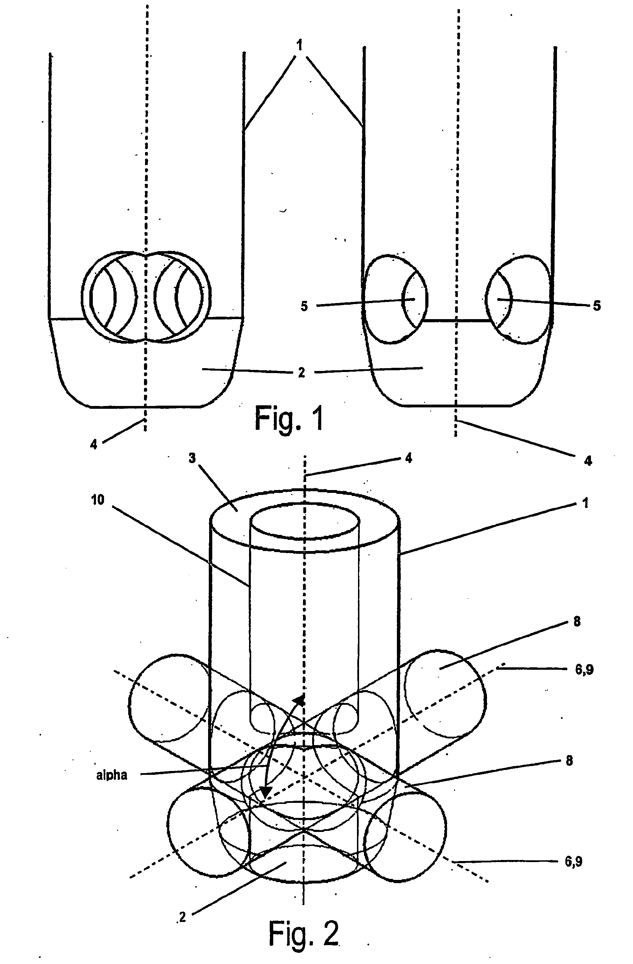

Der in den Fig. 1 bis 3 dargestellte intramedulläre Marknagel weist ein distales, zur Einführung in den Markraum eines Röhrenknochens geeignetes Ende 2 auf, ein proximales Ende 3, eine zentrale Längsachse 4 sowie zwei Querbohrungen 5 mit einer Bohrungsachse 6, welche je einen virtuellen Bohrungszylinder 8 definieren mit einer Zylinderachse 9, welche der Bohrungsachse 6 der definierenden Querbohrung 5 entspricht. Der Marknagel weist einen im Wesentlichen konstanten Durchmesser D auf und eine zur zentralen Längsachse 4 koaxiale Längsbohrung 10.

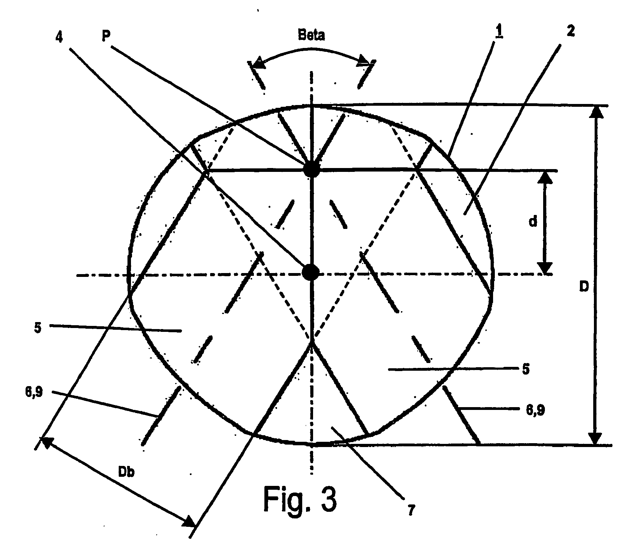

Wie in Fig. 3 dargestellt durchdringen sich die Bohrungszylinder 8 der beiden Querbohrungen 5 gegenseitig, wobei sich die Zylinderachsen 9 der beiden sich durchdringenden Bohrungszylinder 8 im Punkt P schneiden, welcher einen Abstand d = 0,4 D von der Längsachse 4 aufweist. Mit anderen Worten der Punkt P liegt nicht auf der Längsachse 4 des Marknagels.

Die sich durchdringenden Bohrungszylinder 8 weisen zwei getrennte Eingänge in den Marknagel 1 auf, aber nur einen gemeinsamen Ausgang aus dem Marknagel 1 heraus. Die Zylinderachsen 9 der beiden sich durchdringenden Bohrungszylinder 8 liegen in einer zur Längsachse 4 orthogonalen Ebene, welche der Zeichenebene der Fig. 3 entspricht. Die Zylinderachsen 9 der beiden sich durchdringenden Bohrungszylinder 8 können aber auch in einer Ebene liegen, welche von der Längsachse 4 unter einem definierten Winkel α durchstossen wird (Fig. 2).

Im gezeigten Beispiel schneiden die Zylinderachsen 9 der beiden sich durchdringenden Bohrungszylinder 8 unter einem Winkel β von 60°.

Der Durchmesser D_{B} der sich durchdringenden Bohrungszylinder 8 beträgt im gezeigten Beispiel das 0,3-fache von D.

## Patentansprüche

1. Intramedullärer Marknagel (1) mit
A) einem distalen, zur Einführung in den Markraum geeigneten Ende (2), wobei der dem distalen Ende (2) zugewandte Abschnitt des Marknagels (1) einen Durchmesser D aufweist;
B) einem proximalen Ende (3);
C) einer zentralen Längsachse (4); und
D) mehreren Querbohrungen (5) mit einer Bohrungsachse (6), welche je einen virtuellen Bohrungszylinder (8) definieren mit einer Zylinderachse (9), welche der Bohrungsachse (6) der definierenden Querbohrung (5) entspricht, wobei
E) sich die Bohrungszylinder (8) von mindestens zwei Querbohrungen (5) gegenseitig durchdringen; und
F) die Zylinderachsen (9) der beiden sich durchdringenden Bohrungszylinder (8) keinen gemeinsamen Schnittpunkt P auf der Längsachse (4) besitzen,
**dadurch gekennzeichnet, dass**
G) die Zylinderachsen (9) der beiden sich durchdringenden Bohrungszylinder (8) in einer zur Längsachse (4) orthogonalen Ebene liegen.

2. Marknagel (1) nach Anspruch 1, **dadurch gekennzeichnet, dass** sich die Zylinderachsen (9) der beiden sich durchdringenden Bohrungszylinder (8) in einem Schnittpunkt P schneiden, der einen Abstand d > 0 von der Längsachse (4) aufweist.

3. Marknagel (1) nach Anspruch 1 oder 2, **dadurch gekennzeichnet, dass** sich die Zylinderachsen (9) der beiden sich durchdringenden Bohrungszylinder (8) unter einem Winkel β schneiden, wobei 30° < β < 90°, und vorzugsweise 50° < β < 80° ist.

4. Marknagel (1) nach einem der Ansprüche 1 bis 3, **dadurch gekennzeichnet, dass** der Abstand d relativ zum Durchmesser D im Bereich 0,0001 D < d < 0,6000 D, vorzugsweise im Bereich von 0,2 D < d < 0,5 D liegt.

5. Marknagel (1) nach einem der Ansprüche 1 bis 4, **dadurch gekennzeichnet, dass** die sich durchdringenden Bohrungszylinder (8) zwei getrennte Eingänge in den Marknagel (1), aber nur einen gemeinsamen Ausgang aus dem Marknagel (1) heraus aufweisen.

6. Marknagel (1) nach einem der Ansprüche 1 bis 5, **dadurch gekennzeichnet, dass** der Durchmesser D_{B} der sich durchdringenden Bohrungszylinder (8) höchsten das 0,6-fache, vorzugsweise höchstens das 0,5-fache von D beträgt.

7. Marknagel (1) nach einem der Ansprüche 1 bis 6, **dadurch gekennzeichnet, dass** er eine zur zentralen Längsachse (4) koaxiale Längsbohrung (10) aufweist.

## Claims

1. Intramedullary nail (1) with
A) a distal end (2) suitable for insertion into the medullary space, wherein the segment of the intramedullary nail (1) facing the distal end (2) has a diameter D;
B) a proximal end (3);
C) a central longitudinal axis (4); and
D) several cross holes (5) with a hole axis (6), each of which defines a virtual drill cylinder (8) with a cylinder axis (9) corresponding to the hole axis (6) of the defining cross hole (5), wherein
E) the drill cylinders (8) of at least two cross holes (5) penetrate each other,
F) the cylinder axes (9) of the two mutually penetrating drill cylinders (8) have no common intersection point P on the longitudinal axis (4)
**characterised in that**
G) the cylinder axes (9) of the two mutually penetrating drill cylinders (8) lie in a plane orthogonal to the longitudinal axis (4).

2. Intramedullary nail (1) according to claim 1, **characterised in that** the cylinder axes (9) of the two mutually penetrating drill cylinders (8) intersect at an intersection point P, which is at a distance d > 0 from the longitudinal axis (4).

3. Intramedullary nail (1) according to claim 1 or 2, **characterised in that** the cylinder axes (9) of the two mutually penetrating drill cylinders (8) intersect at an angle β, wherein 30° < β < 90°, and preferably 50° < β < 80°.

4. Intramedullary nail (1) according to one of the claims 1 to 3, **characterised in that** the distance d relative to the diameter D lies in the range 0.0001 D < d < 0.6000 D, preferably in the range 0.2 D < d < 0.5 D.

5. Intramedullary nail (1) according to one of the claims 1 to 4, **characterised in that** the mutually penetrating drill cylinders (8) are provided with two separate entrance points into the intramedullary nail (1), but only one common exit from the intramedullary nail (1).

6. Intramedullary nail (1) according to one of the claims 1 to 5, **characterised in that** the diameter D_{B} of the mutually penetrating drill cylinders (8) measures at most 0.6 times, and preferably at most 0.5 times D.

7. Intramedullary nail (1) according to one of the claims 1 to 6, **characterised in that** it is provided with a longitudinal hole (10) coaxial to the central longitudinal axis (4).

## Revendications

1. Clou intramédullaire (1) comprenant :
A) une extrémité distale (2) appropriée à être insérée dans la cavité médullaire, dans lequel la partie du clou intramédullaire (1) dirigée vers l'extrémité distale (2) présente un diamètre D ;
B) une extrémité proximale (3) ;
C) un axe longitudinal central (4) ; et
D) plusieurs alésages transversaux (5) ayant un axe d'alésage (6) définissant chacun un cylindre d'alésage virtuel (8) ayant un axe de cylindre (9), lequel axe correspond à l'axe d'alésage (6) de l'alésage transversal définissant (5), dans lequel
E) les cylindres d'alésage (8) d'au moins deux alésages transversaux (5) se traversent mutuellement ; et
F) les axes de cylindre (9) des deux cylindres d'alésage (8) se traversant mutuellement ne possèdent pas de point d'intersection P commun sur l'axe longitudinal (4),
**caractérisé en ce que**
G) les axes de cylindre (9) des deux cylindres d'alésage (8) se traversant mutuellement se trouvent dans un plan orthogonal à l'axe longitudinal (4).

2. Clou intramédullaire (1) selon la revendication 1, **caractérisé en ce que** les axes de cylindre (9) des deux cylindres d'alésage (8) se traversant mutuellement se coupent au niveau d'un point d'intersection P, lequel présente une distance d > 0 par rapport à l'axe longitudinal (4).

3. Clou intramédullaire (1) selon la revendication 1 ou 2, **caractérisé en ce que** les axes de cylindre (9) des deux cylindres d'alésage (8) se traversant mutuellement se coupent sous un angle β, tel que 30° < β < 90°, et de préférence 50° < β < 80°.

4. Clou intramédullaire (1) selon l'une des revendications 1 à 3, **caractérisé en ce que** la distance d par rapport au diamètre D se situe dans la gamme 0,0001 D < d < 0,6000 D, de préférence dans la gamme 0,2 D < d < 0,5 D.

5. Clou intramédullaire (1) selon l'une des revendications 1 à 4, **caractérisé en ce que** les deux cylindres d'alésage (8) se traversant mutuellement présentent deux entrées séparées dans le clou intramédullaire (1), mais une seule sortie commune hors du clou intramédullaire (1).

6. Clou intramédullaire (1) selon l'une des revendications 1 à 5, **caractérisé en ce que** le diamètre D_{B} des cylindres d'alésage (8) se traversant mutuellement correspond au plus à 0,6 fois, de préférence au plus à 0,5 fois la valeur de D.

7. Clou intramédullaire (1) selon l'une des revendications 1 à 6, **caractérisé en ce qu'**il présente un alésage longitudinal (10) coaxial à l'axe longitudinal central (4).
